Europäisches Patentamt

European Patent Office

Office européen des brevets

(11) Veröffentlichungsnummer: **0 275 957 B1**

(12) **EUROPÄISCHE PATENTSCHRIFT**

(45) Veröffentlichungstag der Patentschrift: 03.03.93

(21) Anmeldenummer: 88100631.6

(22) Anmeldetag: 19.01.88

(51) Int. Cl.5: **C12N 15/31**, A01H 1/00, C12N 5/14, C12N 1/20, //C12N9/10

(54) In Pflanzen wirksames Resistenzgen gegen Phosphinothricin und seine Verwendung.

(30) Priorität: 21.01.87 DE 3701624
07.11.87 DE 3737918

(43) Veröffentlichungstag der Anmeldung:
27.07.88 Patentblatt 88/30

(45) Bekanntmachung des Hinweises auf die Patenterteilung:
03.03.93 Patentblatt 93/09

(84) Benannte Vertragsstaaten:
AT BE CH DE ES FR GB GR IT LI LU NL SE

(56) Entgegenhaltungen:
EP-A- 0 242 246

MOL. GEN. GENET., Band 205, 1986, Seiten 42-50, Springer-Verlag; T. MURAKAMI et al.: "The bialaphos biosynthetic genes of Streptomyces hygroscopicus: Molecular cloning and characterization of the gene cluster"

(73) Patentinhaber: HOECHST AKTIENGESELL-SCHAFT
Postfach 80 03 20
W-6230 Frankfurt am Main 80(DE)

(72) Erfinder: Strauch, Eckhard, Dr.
Rosenheide 2
W-4800 Bielefeld(DE)
Erfinder: Arnold, Walter
Am Gottesberg 25
W-4800 Bielefeld(DE)
Erfinder: Alijah, Renate
Kösterkamp 14
W-4800 Bielefeld(DE)
Erfinder: Wohlleben, Wolfgang, Dr.
Menzelstrasse 1
W-4800 Bielefeld(DE)
Erfinder: Pühler, Alfred, Prof. Dr.
Am Waldschlösschen 2
W-4800 Bielefeld(DE)
Erfinder: Eckes, Peter, Dr.
Am Flachsland 18
W-6233 Kelkheim (Taunus)(DE)

THE EMBO JOURNAL, Band 6, Nr. 9, 1987, Seiten 2519-2523, IRL Press Ltd, Oxford, GB; C.J. THOMPSON et al.: "Characterization of the herbicide-resistance gene bar from Streptomyces hygroscopicus"

THE EMBO JOURNAL, Band 6, Nr. 9, 1987, Seiten 2513-2518, IRL Press Ltd, Oxford, GB; M. DE BLOCK et al.: "Engineering herbicide resistance in plants by expression of a detoxifying enzyme"

CHEMICAL ABSTRACTS, Band 105, 1986, Seite 164, Zusammenfassung Nr. 55584k, Columbus, Ohio, US; J.B. OHLROGGE et al.: "Spinach acyl-carrier proteins: structure, regulation, and progress towards cloning", & BIOCHEM. SOC. TRANS. 1986, 14(3), 579-81

Erfinder: **Donn, Günter, Dr.**
**Sachsenring 35**
**W-6238 Hofheim am Taunus(DE)**
Erfinder: **Uhlmann, Eugen, Dr.**
**Zum Talblick 31**
**W-6246 Glashütten/Taunus(DE)**
Erfinder: **Hein, Friedrich, Dr.**
**Erlesring 40**
**W-6234 Hattersheim am Main(DE)**
Erfinder: **Wengenmayer, Friedrich, Dr.**
**Am Seyenbach 38**
**W-6238 Hofheim am Taunus(DE)**

**Beschreibung**

In der deutschen Patentanmeldung P 36 28 747.4 ist ein Resistenzgen gegen Phosphinothricin (PTC) vorgeschlagen, das aus der Gesamt-DNA von auf Phosphinothricyl-alanylalanin (PTT)-Resistenz selektiertem Streptomyces viridochromogenes DSM 40736 (allgemeine Sammlung) bzw. DSM 4112 (Hinterlegung unter Budapester Vertrag) durch Schneiden mit BamHI, Klonieren eines 4,0 kb großen Fragments und Selektion auf PTT-Resistenz erhältlich ist, sowie die Verwendung dieses Gens zur Herstellung PTC-resistenter Pflanzen, als PTT-Resistenz-Marker in Bakterien und als PTC-Resistenz-Marker in Pflanzenzellen. Das 4 kb große BamHI-Fragment, auf dem das Resistengen liegt, ist durch eine Restriktionskarte (Figur 1) näher definiert.

Durch Klonieren von Teilbereichen dieses 4 kb-Fragmentes wurde die Lage des Codierbereichs näher eingegrenzt. Hierbei zeigte sich, daß das Resistenzgen auf dem 1,6 kb SstII-SstI-Fragment (Positionen 0,55 bis 2,15 in Fig. 1 der Hauptanmeldung) liegt. Durch Verdauung mit BglIII wird ein 0,8 kb großes Fragment gewonnen, das nach Einbau in ein Plasmid und Transformation von S. lividans PTT-Resistenz vermittelt. Diese Resistenz ist durch die N-Acetylierung von PTC bedingt. Das Resistenzgen codiert somit für eine Acetyltransferase.

In der Zusatzanmeldung P 36 42 829.9 ist die DNA-Sequenz des vorstehend genannten 0,8 kb-Fragments wiedergegeben. Aus der Sequenz läßt sich das Startcodon und der offene Leseraster der Gensequenz bestimmen. Das letzte Nucleotid ist Teil des Stop-Codons TGA.

Gene aus Streptomyceten besitzen mit einem Verhältnis Adenin (A) + Thymin (T) : Guanin (G) + Cytosin (C) von ca. 30 % : 70 % einen sehr hohen Anteil an G + C. Der GC-Anteil von Pflanzengenen liegt mit ca. 50 % weitaus niedriger. Aus diesem Grunde wurde in weiterer Ausgestaltung des Erfindungsgedankens die DNA-Sequenz des Resistenzgens durch Neusynthese auf einen für die pflanzliche RNA-Polymerase II günstigen Codongebrauch optimiert.

Die Erfinding betrifft eine Modifikation des Resistenzgens, das in der deutschen Patentanmeldung P 36 28 747.4 und der Zusatzanmeldung P 36 42 829.9 vorgeschlagen ist, nämlich eine Anpassung an den Codongebrauch in Pflanzen. Die entsprechende Aminosäuresequenz ist im Anhang wiedergegeben. Weitere Ausgestaltungen der Erfindung sind in den Patentansprüchen definiert oder werden im folgenden erläutert.

Der genetische Code ist bekanntlich entartet, d.h. daß nur für 2 Aminosäuren ein einziges Triplett codiert, während den restlichen 18 genetisch codierbaren Aminosäuren 2 bis 6 Tripletts zugeordnet sind. Für die Synthese des Gens steht somit theoretisch eine große Vielfalt von Codonkombinationen zur Auswahl. Da der genannte relative Anteil der einzelnen Nukleotide an der Gesamt-DNA-Sequenz von Einfluß ist, wurde er als eines der Kriterien bei der Sequenzoptimierung zugrunde gelegt.

Folgende Änderungen an dem sequenzierten Gen wurden durchgeführt:

1. Das Streptomycetengen-Startcodon GTG (Position 258-260 in der Sequenz der Zusatzanmeldung) wurde gegen das von der pflanzlichen RNA-Polymerase II benutzte Startcodon ATG ausgetauscht.

2. Innerhalb des Gens wurden die Streptomycetengencodons so verändert, daß in Pflanzengenen geeignete Codons daraus resultierten (G/C-Verhältnis).

3. Zur Beendigung des Translationsvorgangs wurde an das Ende der Sequenz das TGA-Stopcodon gesetzt.

4. Anfang und Ende der Gensequenz wurden mit überhängenden Enden von Restriktionsstellen versehen, um das Gen amplifizieren und zwischen pflanzliche Regulationssequenzen ligieren zu können.

5. Palindromische Sequenzen wurden auf ein Mindestmaß reduziert.

Die erfindungsgemäße DNA-Sequenz I (mit der entsprechenden Aminosäuresequenz) ist im Anhang wiedergegeben.

Drei interne singuläre Schnittstellen für die Restriktionsenzyme XbaI (Position 152), BamHI (312) und XmaI (436) ermöglichen die Subklonierung von Teilsequenzen, die in gut untersuchte Klonierungsvektoren, wie etwa pUC18 oder pUC19, eingebaut werden können. Zusätzlich wurden innerhalb des Gens eine Reihe von weiteren singulären Erkennungssequenzen für Restricktionsenzyme eingebaut, die einerseits einen Zugang zu Teilsequenzen der Acetyltransferase schaffen und andererseits die Durchführung von Variationen erlauben:

| Restriktionsenzym | Schnitt nach Nucleotid-Nr. (codierender Strang) |
|---|---|
| BspMII | 11 |
| SacII | 64 |
| EcoRV | 74 |
| HpaI | 80 |
| AatII | 99 |
| BstXI | 139 |
| ApaI | 232 |
| ScaI | 272 |
| AvrII | 308 |
| AflII | 336 |
| StuI | 385 |
| BssHII | 449 |
| FokI | 487 |
| BglI | 536 |
| BglII | 550 |

Der Aufbau der Teilsequenzen durch chemische Synthese und enzymatische Ligationsreaktion erfolgt in an sich bekannter Weise (EP-A 0 133 282, 0 136 472, 0 155 590, 0 161 504, 0 163 249, 0 171 024, 0 173 149 oder 0 177 827). Details wie Restriktionsanalysen, Ligation von DNA-Fragmenten und Transformation von Plasmiden in E. coli sind ausführlich im Lehrbuch von Maniatis (Molecular Cloning, Maniatis et al., Cold Spring Harbor, 1982) beschrieben.

Die so klonierte Gensequenz wird dann unter der Kontrolle pflanzlicher Regulationssignale in Pflanzen eingeführt und zur Expression gebracht. Die EP-A 0 122 791 gibt eine Übersicht über bekannte Methoden. Man erhält so PTC-resistente Pflanzenzellen (d.h. man hat ein Selektionsmerkmal für transformierte Zellen), Pflanzen bzw. Pflanzenteile und Samen.

In den folgenden Beispielen werden einige Ausgestaltungen der Erfindung im einzelnen erläutert. Prozentangaben beziehen sich hierbei auf das Gewicht, wenn nichts anderes angegeben ist.

**Beispiele**

Die folgenden Medien wurden eingesetzt:
a) für Bakterien:

| | |
|---|---|
| YT-Medium: | 0,5 % Hefe-Extrakt, 0,8 % Bacto Trypton, 0,5 % NaCl |
| LB-Medium: | 0,5 % Hefe-Extrakt, 1 % Bacto Trypton, 1 % NaCl |
| als Festmedium: | jeweils Zugabe von 1,5 % Agar |

b) für Pflanzen:

| | |
|---|---|
| M + S-Medium: | Siehe Murashige und Skoog, Physiologica Plantarum 15 (1962) 473 |
| 2MS-Medium: | M + S-Medium mit 2 % Saccharose |
| MSC10-Medium: | M + S-Medium mit 2 % Saccharose, 500 mg/l Cefotaxim, 0,1 mg/l Naphthylessigsäure (NAA), 1 mg/l Benzylaminopurin (BAP), 100 mg/l Kanamycin |
| MSC15-Medium: | M + S-Medium mit 2 % Saccharose, 500 mg/l Cefotaxim, 100 mg/l Kanamycin. |

1. Chemische Synthese eines einzelsträngigen Oligonukleotids

Als Ausgang für die Synthese des Fragments II, eines der vier Teilfragmente I - IV, diente das endständige Oligonukleotid IIc (Nukleotide Nr. 219 bis 312 des codierenden Stranges der DNA-Sequenz I). Für die Festphasensynthese wird das am 3′-Ende stehende Nukleosid, im vorliegenden Fall also Guanosin (Nukleotid Nr. 312), über die 3′-Hydroxyfunktion kovalent an einen Träger gebunden verwendet. Trägermaterial ist mit langkettigen Aminoalkylresten funktionalisiertes CPG ("Controlled Pore Glass"). Im übrigen folgt die Synthese den (aus den auf Seite 4, Zeile 3 - 4 genannten EP-A) bekannten Methoden.

Der Syntheseplan ist in die DNA-Sequenz II (Anhang) eingezeichnet, die im übrigen der DNA-Sequenz I entspricht.

2. Enzymatische Verknüpfung der einzelsträngigen Oligonukleotide zu dem Genfragment II

Zur Phosphorylierung der Oligonukleotide am 5′-Terminus wurde je 1 nmol der Oligonukleotide IIb und IIc mit 5 nmol Adenosintriphosphat und 4 Einheiten T4-Polynukleotid-Kinase in 20 $\mu$l 50 mM Tris-HCl-Puffer (pH 7,6), 10 mM Magnesiumchlorid und 10 mM Dithiothreitol (DTT) 30 Minuten bei 37°C behandelt. Das Enzym wird durch fünfminütiges Erhitzen auf 95°C desaktiviert. Die Oligonukleotide IIa und IId, welche im DNA-Fragment II die "uberhängende" Sequenz bilden, werden nicht phosphoryliert. Dies verhindert bei der nachfolgenden Ligation die Ausbildung größerer Subfragmente als sie dem DNA-Fragment II entsprechen.

Die Oligonukleotide II (a-d) werden wie folgt zum Subfragment II ligiert: Je 1 nmol der Oligonukleotide IIa und IId sowie der 5′-Phosphate von IIb und IIc werden zusammen in 45 $\mu$l Puffer, enthaltend 50 mM Tris-HCl (pH 7,6) 20 mM Magnesiumchlorid, 25 mM Kaliumchlorid und 10 mM DTT, gelöst. Für das "Annealing" der Oligonukleotide gemäß DNA-Fragment II wird die Lösung der Oligonukleotide 2 Minuten auf 95°C erhitzt und dann langsam (2-3 Stunden) auf 20°C abgekühlt. Zur enzymatischen Verknüpfung setzt mann dann 2 $\mu$l 0,1 M DTT, 8 $\mu$l 2,5 mM Adenosintriphosphat (pH 7) sowie 5 $\mu$l T4-DNA-Ligase (2000 Units) zu und inkubiert 16 Stunden bei 22°C.

Die Reinigung des Genfragments II erfolgt durch Gelelektrophorese auf einem 10 %igen Polyacrylamidgel (ohne Harnstoffzusatz, 20 ･ 40 cm, 1 mm Dicke), wobei als Markersubstanz ØX 174 DNA (Fa. BRL), geschnitten mit HinfI, oder pBR322, geschnitten mit HaeIII, diente.

Die Herstellung der Genfragmente I, III und IV erfolgt analog, wobei aber die "überhängenden" Sequenzen vor dem "Annealing" in die 5′-Phosphate übergeführt werden, da kein Ligationsschritt erforderlich ist.

3. Herstellung von Hybridplasmiden, die die Genfragmente I, II, III und IV enthalten.

a) Einbau des Genfragmentes I in pUC18

Das handelsübliche Plasmid pUC18 wird in bekannter Weise mit den Restriktionsendonukleasen SalI und XbaI nach den Angaben der Hersteller geöffnet. Der Verdauungsansatz wird auf einem 1 %igen Agarosegel durch Elektrophorese in bekannter Weise aufgetrennt und die Bruchstücke durch Anfärben mit Ethidiumbromid sichtbar gemacht. Die Plasmidbande (ca. 2,6 kb) wird anschließend aus dem Agarosegel herausgeschnitten und durch Elektroelution von der Agarose abgetrennt.

1 $\mu$g Plasmid, mit XBaI und SalI geöffnet, wird dann mit 10 ng des DNA-Fragments I über Nacht bei 16°C ligiert.

b) Einbau des Genfragmentes II in pUC18.

Analog zu a) wird pUC18 mit XBaI und BamHI aufgeschnitten und mit dem Genfragment II, das zuvor wie in Beispiel 2 beschrieben an den überhängenden Enden phosphoryliert wurde, ligiert.

c) Einbau des Genfragmentes III in pUC18

Analog zu a) wird pUC18 mit BamHI und XmaIII aufgeschnitten und mit dem Genfragment III ligiert.

d) Einbau des Genfragmentes IV in pUC 18

Analog zu a) wird pUC18 mit XmaIII und SalI geschnitten und mit dem Genfragment IV ligiert.

5

4. Aufbau des kompletten Gens und Klonierung in einem pUC-Plasmid

a) Transformation und Amplifikation der Genfragmente I - IV

Die so erhaltenen Hybridplasmide werden in E. coli transformiert. Hierzu wird der Stamm E. coli K 12 durch Behandlung mit einer 70 mM Calciumchloridlösung kompetent gemacht und mit der Suspension des Hybridplasmids in 10 mM Tris-HCl-Puffer (pH 7,5), der 70 mM an Calciumchlorid ist, versetzt. Die transformierten Stämme werden wie üblich unter Ausnutzung der durch das Plasmid vermittelten Antibiotikaresistenzen bzw. -empfindlichkeiten selektioniert und die Hybridvektoren amplifiziert. Nach Abtöten der Zellen werden die Hybridplasmide isoliert, mit den ursprünglich eingesetzten Restriktionsenzymen aufgeschnitten und die Genfragmente I, II, III und IV durch Gelelektrophorese isoliert.

b) Verknüpfung der Genfragmente I, II, III und IV zum Gesamtgen

Die durch Amplifikation erhaltenen Subfragmente I und II werden wie folgt verknüpft. Je 100 ng der isolierten Fragmente I und II werden zusammen in 10 μl Puffer, enthaltend 50 mM Tris-HCl (pH 7,6), 20 mM Magnesiumchlorid und 10 mM DTT, gelöst und diese Lösung wird 5 Minuten auf 57°C erhitzt. Nachdem die Lösung auf Raumtemperatur abgekühlt ist, gibt man 1 μl 10 mM Adenosintriphosphat (pH 7) sowie 1 μl T4-DNA-Ligase (400 Units) zu und inkubiert 16 Stunden bei Raumtemperatur. Nach dem Nachschneiden mit den Restricktionsenzymen SalI und BamHI wird das gewünschte 312 bp-Fragment (Nukleotide 1-312, SalI-BamHI) durch Gelelektrophorese auf einem 8 %igen Polyacrylamidgel gereinigt, wobei als Markersubstanz ØX 174 RF DNA (Fa. BRL), geschnitten mit dem Restriktionsenzym HaeIII, dient.

In gleicher Weise werden die Genfragmente III und IV miteinander verknüpft, wobei man nach der Reinigung ein 246 bp-Fragment (Nukleotide 313-558, BamHI-SalI) erhält. Als Marker bei der Gelelektrophorese wird pBR322, geschnitten mit dem Restriktionsenzym MspI, verwendet.

Zum Aufbau des Gesamtgens (DNA-Sequenz I) werden 15 ng des 312 bp-Fragmentes und 12 ng des 245bp-Fragmentes mit 1 μg des handelsüblichen Plasmids pUC18, das zuvor mit dem Restriktionsenzym SalI aufgeschnitten und an den Enden enzymatisch dephosphoryliert wurde, wie oben beschrieben ligiert. Nach der Transformation und Amplifikation (wie in Beispiel 4a) beschrieben) wird durch SalI-Verdauung der richtige Klon mit dem 558 bp-Fragment gemäß der DNA-Sequenz I identifiziert.

5. Transformation der Hybridplasmide

Kompetente E. coli-Zellen werden mit 0,1 bis 1 μg des Hybridplasmids, das die DNA-Sequenz I enthält, transformiert und auf Ampicillin enthaltenden Agarplatten plattiert. Anschließend lassen sich Klone, die die korrekt integrierten Sequenzen im Plasmid enthalten, durch DNA-Schnellaufarbeitung identifizieren (Maniatis a.a.O.).

6. Fusion des synthetisierten Gens an Regulationssignale, die in Pflanzen erkannt werden.

Das an den Enden mit SalI-Schnittstellen versehene optimierte Resistenzgen wurde in die SalI-Schnittstelle der Polylinkersequenz des Plasmides pDH51 (Pietrzak et al., Nucleic Acids Res. 14 (1986) 5857) ligiert. Auf diesem Plasmid sind Promotor und Terminator des 35S-Transkripts aus Cauliflower Mosaic Virus lokalisiert, die vom pflanzlichen Transkriptionsapparat erkannt werden.

Durch die Ligation des Resistenzgens wurde dieses hinter dem Promotor und vor dem Terminator des 35S-Transkripts inseriert. Die korrekte Orientierung des Gens wurde durch Restriktionsanalysen bestätigt.

Der Promotor des ST-LS1-Gen aus Solanum tuberosum (Eckes et al., Mol. Gen. Genet. 205 (1986) 14) wurde ebenfalls zur Expression des optimierten Acetyltransferase-Gens in Pflanzen verwendet.

7. Einschleusen des Resistenzgens mit den Regulationssequenzen in Agrobacterium tumefaciens

a) Kointegrat-Methode

Die gesamte Transkriptionseinheit aus Promotor, optimiertem Resistenzgen und Terminator (Beispiel 6) wurde mit dem Restriktionsenzym EcoRI ausgeschnitten und in die EcoRI-Schnittstelle des intermediären E. coli-Vektors pMPK110 ligiert (Peter Eckes, Dissertation, Univ. Köln, 1985, S. 91 f.). Dieser intermediäre Vektor war nötig, um das Resistenzgen mit seinen Regulationssequenzen in das Ti-Plasmid von Agrobacterium tumefaciens zu überführen. Diese sogenannte Konjugation wurde nach dem von Van Haute et al.

(EMBO J. 2 (1983) 411) beschriebenen Verfahren durchgeführt. Dabei wurde das Gen mit seinen Regulationssignalen durch homologe Rekombination über die im pMPK110-Vektor und im Ti-Plasmid pGV3850kanR (Jones et al., EMBO J. 4 (1985) 2411) enthaltenen Sequenzen des Standardvektors pBR322 in das Ti-Plasmid integriert.

Dazu wurden je 50 μl frische Flüssigkulturen der E. coli-Stämme DH1 (Wirtsstamm des pMPK110-Derivates) und GJ23 (Van Haute et al., Nucleic Acids Res. 14 (1986) 5857) auf einer trockenen YT-Agarplatte vermischt und für eine Stunde bei 37°C inkubiert. Die Bakterien wurden in 3 ml 10 mM MgSO₄ resuspendiert und auf Antibiotika-Agarplatten ausplattiert (Spectinomycin 50 μg/ml: Selektion auf pMPK110; Tetracyclin 10 μg/ml: Selektion auf R64drd11; Kanamycin 50 μg/ml: Selektion auf pGJ28). Die auf den selektiven Agarplatten wachsenden Bakterien enthielten die drei Plasmide und wurden für die Konjugation mit Agrobacterium tumefaciens in YT-Flüssigmedium bei 37°C vermehrt. Die Agrobakterien wurden in LB-Medium bei 28°C kultiviert. Je 50 μl Bakteriensuspension wurden auf einer trockenen YT-Agarplatte vermischt und für 12 bis 16 Stunden bei 28°C inkubiert. Die Bakterien wurden in 3 ml 10 mM MgSO₄ resuspendiert und auf Antibiotikaplatten ausplattiert (Erythromycin 0,05 g/l, Chloramphenicol 0,025 g/l: Selektion auf den Agrobakterienstamm; Streptomycin 0,3 g/l und Spectinomycin 0,1 g/l: Selektion auf die Integration des pMPK110 in das Ti-Plasmid). Auf diesen selektiven Platten können nur Agrobakterien wachsen, bei denen das pMPK110-Derivat durch eine homologe Rekombination in das bakterielle Ti-Plasmid integriert ist.

Auf dem Ti-Plasmid pGV3850kanR war nun neben dem schon vorher vorhandenen, in Pflanzen wirksamen Resistenzgen gegen das Antibiotikum Kanamycin auch das Resistenzgen gegen PTC lokalisiert. Bevor diese Agrobakterienklone für die Transformation eingesetzt wurden, wurde durch ein "Southern-Blot"-Experiment überprüft, ob die gewünschte Integration erfolgt war.

b) Binäre Vektor-Methode

Es wurde das binäre Vektrosystem, das von Koncz et al. (Mol. Gen. Genet. 204 (1986) 383) beschrieben worden war, verwendet. Der von Koncz et al. (PNAS 84 (1987) 131) beschriebene Vektor pPCV701 wurde folgendermaßen verändert: Mit den Restriktionsenzymen BamHI und HindIII wurde ein Fragment, auf dem u.a. die Promotoren TR1 und TR2 lokalisiert sind, aus dem Vektor entfernt. Das resultierende Plasmid wurde rezirkularisiert. In die vorhandene EcoRI-Schnittstelle auf diesem Vektor wurde ein ca. 800 Basenpaare langes Fragment aus dem Vektor pDH51 inseriert, auf dem Promotor und Terminator des 35S-Transkriptes aus Cauliflower Mosaic Virus lagen (Pietrzak et al., Nucleic Acids Res. 14 (1986) 5858). Das resultierende Plasmid pPCV801 hatte zwischen 35S-Promotor und -Terminator eine singuläre SalI-Schnittstelle. In diese Schnittstelle wurde das optimierte PTC-Resistenzgen inseriert. Seine Expression stand nun unter der Kontrolle der 35S-Transkript-Regulationssequenzen.

Dieses Plasmid (pPCV801Ac) wurde in den E. coli-Stamm SM10 transformiert (Simon et al., Bio/Technology 1 (1983), 784). Zur Überführung des Plasmids pPCV801Ac nach Agrobacterium tumefaciens wurden je 50 μl der SM10-Kultur und einer C58-Agrobakterienkultur (GV3101, Van Larebeke et al., Nature 252 (1974) 169) mit dem Ti-Plasmid pMP90RK (Koncz et al., Mol. Gen. Genet. 204 (1986) 383) als Helferplasmid auf einer trockenen YT-Agarplatte vermischt und für ca. 16 Stunden bei 28°C inkubiert. Die Bakterien wurden anschließend in 3 ml 1 mM MgSO₄ resuspendiert und auf Antibiotikaplatten ausplattiert (Rifampicin 0,1 g/l: Selektion auf GV3101, Kanamycin 0,025 g/l: Selektion auf pMP90RK, Carbenicillin 0,1 g/l: Selektion auf pPCV801Ac). Auf diesen Platten können nur Agrobakterien wachsen, die beide Plasmide (pMP90RK und pPCV801Ac) enthalten. Bevor diese Agrobakterien für die Pflanzentransformation eingesetzt wurden, wurde durch "Southern Blotting" überprüft, daß das Plasmid pPCV801Ac in seiner korrekten Form in den Agrobakterien vorhanden ist.

8. Transformation von Nicotiana tabacum durch Agrobacterium tumefaciens

Das optimierte Resistenzgen wurde mittels der sogenannten "leaf disc" Transformationsmethode in Tabakpflanzen übertragen.

Die Agrobakterien wurden in 30 ml LB-Medium mit den entsprechenden Antibiotika bei 28°C unter ständigem Schütteln herangezogen (etwa 5 Tage). Dan wurden die Bakterien durch eine zehnminütige Zentrifugation bei 7000 rpm in einer Christ-Zentrifuge sedimentiert und einmal mit 20 ml 10 mM MgSO₄ gewaschen. Nach einer weiteren Zentrifugation wurden die Bakterien in 20 ml 10 mM MgSO₄ suspendiert und in eine Petrischale überführt. Für die Blattscheiben-Infektion wurden Blätter von in Sterilkultur auf 2MS-Medium wachsenden Wisconsin 38-Tabakpflanzen verwendet. Alle Sterilkulturen wurden bei 25 bis 27°C in einem 16 Stunden Licht/8 Stunden Dunkel-Rhythmus bei Weißlicht gehalten.

Tabakblätter wurden abgeschnitten und die Blattoberflächen mit Sandpapier verwundet. Nach der Verwundung wurden die Blätter in kleinere Stücke zerschnitten und in die Bakterienkultur getaucht. Dann wurden die Blattstücke auf M + S-Medium transferiert und für zwei Tage unter normalen Kulturbedingungen gehalten. Nach der zweitägigen Infektion mit den Bakterien wurden die Blattstücke in flüssigem M + S-Medium gewaschen und auf MSC10-Agarplatten überführt. Transformierte Sprosse wurden aufgrund der mitübertragenen Resistenz gegen das Antibiotikum Kanamycin selektioniert. 3 bis 6 Wochen später wurden die ersten Sprosse sichtbar. Einzelene Sprosse wurden auf MSC15-Medium in Glasdosen weiterkultiviert. In den folgenden Wochen bildeten einige der abgeschnittenen Sprosse an der Schnittstelle Wurzeln.

Transformierte Pflanzen konnten auch direkt auf PTC-haltigen Pflanzenmedien selektioniert werden. Durch DNA-Analyse ("Southern Blotting") und RNA-Analyse ("Northern Blotting") der transformierten Pflanzen wurde das Vorhandensein und die Expression des PTC-Resistenzgens nachgewiesen.

9. Nachweise der PTC-Resistenz der transformierten Pflanzen

Zur Überprüfung der Funktionalität des Resistenzgens in transformierten Pflanzen wurden Blattfragmente transformierter und nichttransformierter Pflanzen auf M + S-Nährmedien mit $1.10^{-4}$ M L-PTC überführt. Die Fragmente nichttransformierter Pflanzen starben ab, während aus den Fragmenten transformierter Pflanzen neue Sprosse regeneriert werden konnten. Transformierte Sprosse bewurzelten und wuchsen ohne Probleme auf M + S-Nährmedien mit $1.10^{-3}$ M L-PTC. Transformierte Pflanzen wurden aus sterilen Bedingungen in Erde getopft und mit 2 kg/ha und 5 kg/ha PTC besprüht. Während nichttransformierte Pflanzen diese Herbizidbehandlung nicht überlebten, zeigten transformierte Pflanzen keine durch das Herbizid bewirkten Schädigungen. Das Aussehen und Wuchsverhalten der besprühten, transformierten Pflanzen war mindestens genauso gut wie bei unbesprühten Kontrollpflanzen.

10. Acetyltransferase-Test zum Nachweis der PTC-Acetylierung in transgenen, PTC-resistenten Pflanzen

Ca. 100 mg Blattgewebe von transgenen, PTC-resistenten Tabakpflanzen bzw. von nichttransformierten Tabakpflanzen wurden in einem Puffer, bestehend aus: 50 mM Tris/HCl pH 7,5; 2 mM EDTA; 0,1 mg/ml Leupeptin; 0,3 mg/ml Rinderserumalbumin; 0,3 mg/ml DTT; 0,15 mg/ml Phenylmethylsulfonylfluorid (PMSF) homogenisiert.

Nach anschließender Zentrifugation wurden 20 $\mu$l des klaren Überstandes mit 1 $\mu$l 10 mM radioaktiv markierten D,L-PTC und 1 $\mu$l 100 mM Acetyl-CoA für 20 Minuten bei 37°C inkubiert. Anschließend wurde die Reaktionsmischung mit 25 $\mu$l 12 % Trichloressigsäure versetzt und abzentrifugiert. Vom Überstand wurden 7 $\mu$l auf eine Dünnschichtchromatographie-Platte übertragen und in einem Gemisch aus Pyridin : n-Butanol : Essigsäure : Wasser (50 : 75 : 15 : 60 Volumenteile) aufsteigend zweimal entwickelt. PTC und Acetyl-PTC wurden so voneinander getrennt und konnten durch Autoradiographie nachgewiesen werden. Nichttransformierte Pflanzen zeigten keine Umsetzung des PTC zum Acetyl-PTC, während transgene, resistente Pflanzen dazu in der Lage waren.

```
                                                                                  1
TC GAC  MET SER PRO GLU ARG ARG PRO VAL ILE GLU ILE ARG PRO ALA THR ALA ALA ASP MET ALA ALA VAL CYS ASP ILE VAL ASN HIS TYR
 G      ATG TCT CCG GAG AGG AGA CCA GTT ATT GAG ATC AGG CCA GCA ACA GCT GCC GAT ATG GCA GCG GTT TGT GAT ATC GTT AAC CAT TAC
        TAC AGA GGC CTC TCC TCT GGT CAA TAA CTC TAG TCC GGT CGT TGT CGA CGG CTA TAC CGT CGC CAA ACA CTA TAG CAA TTG GTA ATG
                                                                    100

        ILE GLU SER THR VAL ASN PHE ARG GLU THR PRO GLN GLU TRP ILE ASP ARG LEU GLU ARG LEU GLN ASP ARG TYR PRO
        ATT GAG TCT ACA GTG AAC TTT AGG GAG ACA CCA CAA GAG TGG ATT GAT AGG CTA GAG AGG TTG CAA GAT AGA TAC CCT
        TAA CTC AGA TGT CAC TTG AAA TCC CTC TGT GGT GTT CTC ACC TAA CTA TCC GAT CTC TCC AAC GTT CTA TCT ATG GGA
                                                                                200

        TRP LEU VAL ALA GLU VAL GLY VAL ALA GLY ILE ALA TYR ALA GLY PRO TRP LYS ALA ARG ASN ALA TYR ASP TRP THR VAL GLU
        TGG TTG GTT GCT GAG GTT GGT GTG GCT GGT ATT GCT TAC GCT GGG CCC TGG AAG GCT AGG AAC GCT TAC GAT TGG ACA GTT GAG
        ACC AAC CAA CGA CTC CAA CCA CAC CGA CCA TAA CGA ATG CGA CCC GGG ACC TTC CGA TCC TTG CGA ATG CTA ACC TGT CAA CTC
                                                                                300

        SER THR VAL TYR VAL SER HIS ARG HIS GLN ARG LEU GLY LEU LEU TYR THR HIS LEU LYS SER MET GLU ALA GLN GLY
        AGT ACT GTT TAC GTG TCA CAT AGG CAT CAA AGG CTT GGC CTA TTG TAC ACA CAT CTT AAG TCT ATG GAG GCG CAA GGT
        TCA TGA CAA ATG CAC AGT GTA TCC GTA GTT TCC GAA CCG GAT AAC ATG TGT GTA GAA TTC AGA TAC CTC CGC GTT CCA
                                                                                400

        PHE LYS SER VAL VAL ALA VAL ILE GLY LEU PRO ASN ASP PRO SER VAL ARG LEU HIS GLU ALA ARG GLY TYR THR LEU
        TTT AAG TCT GTT GTT GCT GTT ATA GGC CTT CCA AAC GAT CCA TCT GTT AGG CTT CAT GAG GCT AGG GGA TAC ACA TTG
        AAA TTC AGA CAA CAA CGA CAA TAT CCG GAA GGT TTG CTA GGT AGA CAA TCC GAA GTA CTC CGA TCC CCT ATG TGT AAC
                                                                            500

        ARG ALA ALA GLY TYR LYS HIS GLY TRP HIS ASP VAL VAL GLN ASP PHE GLU LEU PRO ALA PRO PRO VAL ARG
        CGC GCA GCT GGA TAC AAG CAT GGT TGG CAT GAT GTT GTT CAA GGG GAT TTT GAG CCT GCT CCA CCA GTT AGG
        GCG CGT CGA CCT ATG TTC GTA CCA ACC GTA CTA CAA CAA GTT CCC CTA AAA CTC GGA CGA GGT GGT CAA TCC

        PRO VAL THR GLN ILE ---
        CCA GTT ACC CAG ATC TGA G
        GGT CAA TGG GTC TAG ACT CAG CT
```

Aminosäure- und DNA-Sequenz I

```
Aminosäure- und DNA-Sequenz II

Ia / Ib
MET SER PRO GLU ARG ARG PRO VAL GLU ILE ARG PRO ALA THR ALA ALA VAL CYS ASP ILE VAL ASN HIS TYR
ATG TCT CCG GAG AGG AGA CCA GTT GAG ATT AGG CCA GCT ACA GCT GCA GTT TGT GAT ATC GTT AAC CAT TAC
TAC AGA GGC CTC TCC TCT GGT CAA CTC TAA TCC GGT CGA TGT CGA CGT CAA ACA CTA TAG CAA TTG GTA ATG
 TC GAC                                                                                          
  G TAC

(Fragmente: Ia, Ib, Ic, Id, IIa, IIb, IIc, IId, IIIa, IIIb, IVa, IVb)

... ILE GLU THR SER VAL ASN PHE ARG ... ASP ARG TYR PRO ... GLU ARG LEU ASP ...
... TRP LEU VAL ALA GLY VAL VAL ALA GLY PRO TRP LYS ALA ARG ASN ALA TYR ... ASP TRP THR VAL GLU ...
... SER THR VAL TYR VAL HIS ARG SER TCA ... LYS SER MET GLU GLY ALA GLN GLY ...
... ALA ALA GLY TYR LYS HIS GLY GLY TRP HIS ASP VAL GLY VAL ... ALA ARG GLY TYR THR LEU ...
... ARG ALA ALA GLY TYR LYS HIS ... PRO VAL THR GLN ILE ... PRO PRO ARG PRO VAL ARG ...
... PRO VAL THR GLN ILE ---
```

**Patentansprüche für folgende Vertragsstaaten : AT, BE, CH, DE, FR, GB, GR, IT, LI, LU, NL, SE**

1. Resistenzgen, codierend für das Protein der Aminosäuresequenz I (Anhang), indem als Startcodon ATG und als Stopcodon TGA verwendet werden und der GC-Anteil des Gens an den in Pflanzen angepaßt ist.

2. Resistenzgen nach Anspruch 1, gekennzeichnet durch die DNA-Sequenz I (Anhang, Nukleotidposition 9-554).

3. Genstruktur, gekennzeichnet durch die DNA-Sequenz I (Anhang), gekoppelt an in Pflanzen wirksame Regulations-und Expressionssignale.

4. Vektor, gekennzeichnet durch das Resistenzgen nach Anspruch 1 oder 2.

5. Vektor, gekennzeichnet durch eine Genstruktur nach Anspruch 3.

6. Vektoren, enthaltend eine oder mehrere DNA-Sequenzen (Anhang) aus der

Gruppe I     (Nucleotid No.    1 bis 152),

II     ("          "   153 "   312),

III     ("          "   313 "   436) oder

IV     ("          "   437 "   558).

7. Wirtszelle, gekennzeichnet durch einen Vektor nach Anspruch 4, 5 oder 6.

8. Pflanzenzelle, gekennzeichnet durch ein Gen nach Anspruch 1, 2 oder 3.

9. Pflanzen, deren Teile und Samen, gekennzeichnet durch ein Gen nach Anspruch 1, 2 oder 3.

10. Verwendung des Gens Anspruch 1 oder 2 bzw. der Genstruktur nach Anspruch 3 zur Erzeugung von Phosphinothricin-resistenten Pflanzenzellen, Pflanzenteilen, Pflanzen und Samen.

**Patentansprüche für folgenden Vertragsstaat : ES**

1. Verfahren zur Herstellung eines Resistenzgens gegen Phosphinothricin (PTC), dadurch gekennzeichnet, daß man ein Gen synthetisiert, das für das Protein der Aminosäuresequenz I (Anhang) codiert, wobei als Startcoden ATG und als Stopcodon TGA verwendet werden und der GC-Anteil des Gens an den in Pflanzen angepaßt ist.

2. Verfahren nach Anspruch 1, dadurch gekennzeichnet, daß das Gen die DNA-Sequenz I (Anhang, Nucleotidposition 9-554) hat.

3. Verfahren zur Herstellung PTC-resistenter Pflanzenzellen, Pflanzen, Pflanzenteile oder Samen, dadurch gekennzeichnet, daß man ein nach Anspruch 1 oder 2 erhaltenes Gen an in Pflanzen wirksame Regulations- und Expressionssignale koppelt, die so erhaltene Genstruktur in Pflanzenzellen einbringt und darin zur Expression bringt.

**Claims**
**Claims for the following Contracting States : AT, BE, CH, DE, FR, GB, GR, IT, LI, LU, NL, SE**

1. A resistance gene coding for the protein of amino acid sequence I (annex), in that ATG is used as start codon and TGA is used as stop codon, and the GC content of the gene is adapted to that in plants.

2. The resistance gene as claimed in claim 1, having DNA sequence I (annex, nucleotide position 9-554).

3. A gene structure having DNA sequence I (annex) coupled to regulation and expression signals active in plants.

**4.** A vector containing the resistance gene as claimed in claim 1 or 2.

**5.** A vector containing a gene structure as claimed in claim 3.

**6.** Vectors containing one or more DNA sequences (annex) from

| Group I | (nucleotide No. 1 to 152), |
|---|---|
| Group II | (nucleotide No. 153 to 312), |
| Group III | (nucleotide No. 313 to 436) or |
| Group IV | (nucleotide No. 437 to 558). |

**7.** A host cell containing a vector as claimed in claim 4, 5 or 6.

**8.** A plant cell containing a gene as claimed in claim 1, 2 or 3.

**9.** Plants, their parts and seeds containing a gene as claimed in claim 1, 2 or 3.

**10.** The use of the gene as claimed in claim 1 or 2 or of the gene structure as claimed in claim 3 for generating phosphinothricin-resistant plant cells, parts of plants, plants and seeds.

**Claims for the following Contracting State : ES**

**1.** A process for the preparation of a phosphinothricin (PTC)-resistance gene, which comprises synthesis of a gene which codes for the protein of amino acid sequence I (annex), wherein ATG is used as start codon and TGA is used as stop codon, and the GC content of the gene is adapted to that in plants.

**2.** The process as claimed in claim 1, wherein the gene has DNA sequence I (annex, nucleotide position 9-554).

**3.** A process for the generation of PTC-resistant plant cells, plants, parts of plants or seeds, which comprises coupling a gene which has been obtained as claimed in claim 1 or 2 to regulation and expression signals active in plants, introducing the resulting gene structure into plant cells, and bringing about its expression therein.

**Revendications**
**Revendications pour les Etats contractants suivants : AT, BE, CH, DE, FR, GB, GR, IT, LI, LU, NL, SE**

**1.** Gène de résistance, codant pour la protéine de la séquence d'acides aminés I (annexe), obtenu par utilisation d'ATG en tant que codon initiateur et de TGA en tant que codon de terminaison, la partie GC du gène étant adaptée à celle des végétaux.

**2.** Gène de résistance selon la revendication 1, caractérisé par la séquence d'ADN I (annexe, position des nucléotides 9-554).

**3.** Structure génique, caractérisée par la séquence d'ADN I (annexe), couplée à des signaux de régulation et d'expression actifs dans les végétaux.

**4.** Vecteur caractérisé par le gène de résistance selon la revendication 1 ou 2.

**5.** Vecteur caractérisé par une structure génique selon la revendication 3.

**6.** Vecteurs contenant une ou plusieurs séquences d'ADN (annexe) provenant du

| groupe | I | (nucléotides n° 1 à 152), |
|--------|-----|----------------------------|
| | II | (nucléotides n° 153 à 312), |
| | III | (nucléotides n° 313 à 436) ou |
| | IV | (nucléotides n° 437 à 558). |

**7.** Cellule hôte caractérisée par un vecteur selon la revendication 4, 5 ou 6.

**8.** Cellule végétale caractérisée par un gène selon la revendication 1, 2 ou 3.

**9.** Végétaux,leurs éléments ou parties et graines, caractérisés par un gène selon la revendication 1, 2 ou 3.

**10.** Utilisation du gène selon la revendication 1 ou 2, ou de la structure génique selon la revendication 3, pour produire des cellules végétales, des parties de végétaux, des végétaux ou des graines résistant à la phosphinotricine.

**Revendications pour l'Etat contractant suivant : ES**

**1.** Procédé de préparation d'un gène conférant la résistance à la phosphinotricine (PTC), caractérisé en ce qu'on synthétise un gène qui code pour la protéine ayant la séquence d'acides aminés I (annexe), en utilisant l'ATG comme codon initiateur et le TGA code codon de terminaison, et en ce que la partie GC du gène est adaptée à celle des végétaux.

**2.** Procédé selon la revendication 1, caractérisé en ce que le gène a la séquence d'ADN I (annexe, position des nucléotides 9-554)

**3.** Procédé de préparation de cellules végétales, de végétaux, de parties de végétaux ou de graines résistant au PTC, caractérisé en ce qu'on couple un gène obtenu selon la revendication 1 ou 2 à des signaux de régulation et d'expression actifs dans les végétaux, on introduit la structure génique ainsi obtenue dans des cellules végétales et on en provoque l'expression dans ces cellules.